(19) Europäisches Patentamt
European Patent Office
Office européen des brevets

(11) Veröffentlichungsnummer: **0 052 744 B1**

# (12) EUROPÄISCHE PATENTSCHRIFT

(45) Veröffentlichungstag der Patentschrift: **12.10.83**

(51) Int. Cl.³: **C 07 C 131/00**

(21) Anmeldenummer: **81108264.3**

(22) Anmeldetag: **13.10.81**

## (54) Verfahren zur Herstellung von alpha-Bromacetophenonoximethern.

(30) Priorität: **26.11.80 DE 3044564**

(43) Veröffentlichungstag der Anmeldung: **02.06.82 Patentblatt 82/22**

(45) Bekanntmachung des Hinweises auf die Patenterteilung: **12.10.83 Patentblatt 83/41**

(84) Benannte Vertragsstaaten: **BE DE FR GB IT**

(56) Entgegenhaltungen:
**THE JOURNAL OF ORGANIC CHEMISTRY, Band 36, Nr. 22, 5. November 1971, Seiten 3467–3469 Washington D.C., U.S.A. SOU-YIE CHU et al.: "Preparation of 2-alkoxyiminoalkyl bromides by the bromination of O-alkyl oximes with N-bromosuccinimide"**

(73) Patentinhaber: **BASF Aktiengesellschaft, Carl-Bosch-Strasse 38, D-6700 Ludwigshafen (DE)**

(72) Erfinder: **Schaefer, Peter, Dr., Im Buegen 16, D-6719 Kirchheim (DE)**
Erfinder: **Mangold, Dietrich, Dr., Hermann-Walker-Strasse 49, D-6903 Neckargemuend (DE)**

EP 0 052 744 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von α-Bromacetophenonoximethern

Die vorliegende Erfindung betrifft ein neues Verfahren zur Herstellung von α-Bromacetophenonoximethern durch Umsetzung von Acetophenonoximethern mit einem Bromierungsmittel unter Lichtausschluss bei 20 bis 100°C.

Es ist bekannt, dass man Ketoxim-O-ether oder Aldoxim-O-ether durch Reaktion mit N-Bromsuccinimid unter Belichtung in α-Position bromieren kann (J. org. Chem. 36, 3467 bis 3469 (1971)). Nach diesem Verfahren sind jedoch nur O-Alkyloximether zugänglich, da bei anderen Substituenten am Oximsauerstoffatom, z.B. Benzyl, Angriff von Brom an diesem Substituenten erfolgt unter Bildung von Substanzgemischen. Die Wichtigkeit einer guten Belichtung (UV-Licht) wird hervorgehoben.

Es wurde nun gefunden, dass man α-Bromacetophenonoximether der Formel

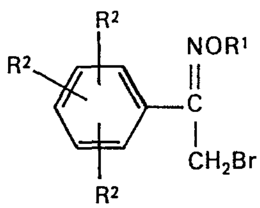
I,

worin $R^1$ einen araliphatischen oder zweifach ungesättigten aliphatischen Rest bedeutet, die einzelnen Reste $R^2$ gleich oder verschieden sein können und jeweils ein Wasserstoffatom, ein Halogenatom oder einen aliphatischen Rest bezeichnen, vorteilhaft erhält, wenn man Acetophenonoximether der Formel

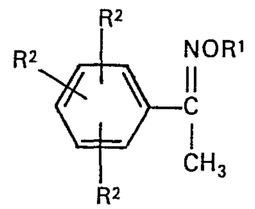
II,

worin $R^1$ und $R^2$ die vorgenannten Bedeutungen besitzen, mit einem Bromierungsmittel bei einer Temperatur von 20 bis 100°C unter Lichtausschluss umsetzt.

Die Umsetzung kann für den Fall der Verwendung von 2,4-Di-chloracetophenonoxim-O-benzylether und Brom durch die folgenden Formeln wiedergegeben werden:

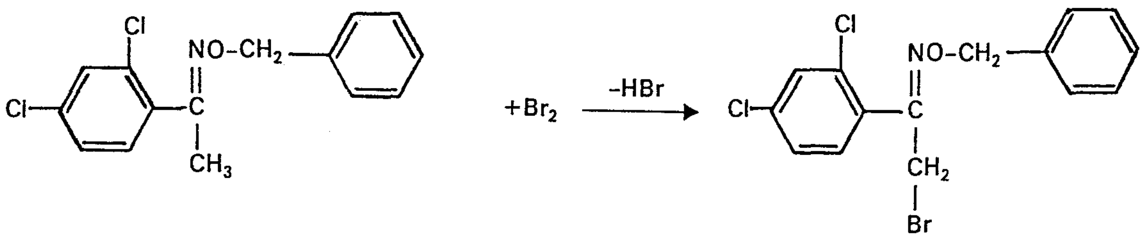


Im Hinblick auf den Stand der Technik liefert das Verfahren nach der Erfindung überraschend auf einfacherem und wirtschaftlicherem Wege α-Bromacetophenonoximether in besserer Ausbeute, Raum-Zeit-Ausbeute und Reinheit, gerade auch im grosstechnischen Massstab und im kontinuierlichen Betrieb.

Es ist überraschend, dass bei den erfindungsgemässen Verfahrensbedingungen die Acetophenonoximether II stereoselektiv an der $-CH_3$-Gruppe bromiert werden. Angriff an $R^1$ ist unerwarteterweise nicht zu beobachten, obwohl eine Aktivierung der Wasserstoffatome dieses Molekülteils durch den Aromaten bzw. die C–C-Dreifachbindung gegeben ist (Houben-Weyl, Methoden der Organischen Chemie, Band 5/4, Seiten 153–163). Ferner treten keine Spaltreaktionen durch den im Laufe der Reaktion gebildeten Bromwasserstoff auf.

Das erfindungsgemässe Verfahren weist eine Reihe von Vorteilen auf. Auch α-Bromketoximether sind zugänglich, deren Substituenten am Oximsauerstoff aktivierte Wasserstoffatome tragen. Diese Wasserstoffatome werden unter den erfindungsgemässen Verfahrensbedingungen nicht durch Brom substituiert. Ferner kann elementares Brom als Halogenierungsreagenz eingesetzt werden. Radikalstarter oder Belichtung sind nicht erforderlich. Alle diese vorteilhaften Ergebnisse sind im Hinblick auf den Stand der Technik überraschend. Auch hätte vermutet werden müssen, dass der Verzicht auf starke Lichtquellen bei einer Bromierung zu erhöhten Ausbeuteminderungen, wenn nicht gar Blockierung der Reaktion führen würde.

Der Ausgangsstoff II kann mit dem Bromierungsmittel in stöchiometrischer Menge oder im Überschuss, vorzugsweise in einem Verhältnis von 1 bis 1,25, insbesondere 1 bis 1,1 Mol Bromierungsmittel je Mol Ausgangsstoff II, umgesetzt werden. Bevorzugte Ausgangsstoffe II und dementsprechend bevorzugte Endstoffe I sind solche, in deren Formeln $R^1$ einen Alkinylrest mit 2 bis 20, insbesondere 2 bis 8 Kohlenstoffatomen, oder

einen Aralkylrest mit 7 bis 12 Kohlenstoffatomen bedeutet, die einzelnen Reste $R^2$ gleich oder verschieden sein können und jeweils ein Wasserstoffatom, ein Bromatom, ein Jodatom oder insbesondere ein Chloratom oder einen Alkylrest mit 1 bis 6 Kohlenstoffatomen bezeichnen. Die vorgenannten Reste können noch durch unter den Reaktionsbedingungen inerten Gruppen und Atomen, z.B. Chloratome, Bromatome, Alkylgruppen mit 1 bis 4 Kohlenstoffatomen, substituiert sein.

So kommen beispielsweise als Ausgangsstoffe II in Betracht: Benzyl-, Ethinyl-, Propinyl-, 1-Butinyl-, Pentinyl-, Propargyl-, 2-Butinyl-, Phenylethyl-, Phenylpropyl-O-ether des Acetophenonoxims. Bevorzugt sind der 2,4-Dichloracetophenonoxim-O-propargylether und der 2,4-Dichloracetophenonoxim-O-benzylether.

Als Bromierungsmittel kommen im allgemeinen Brom oder unter den Reaktionsbedingungen Brom bildende Stoffe, im allgemeinen Säurebromide, in Betracht. Als Brom bildende Stoffe kommen vorteilhaft Bromide zusammen mit einem Oxidationsmittel und Säure in Betracht; ebenfalls kann man Oxidationsmittel und Bromwasserstoff, vorteilhaft in Gestalt der wässrigen Bromwasserstofflösung, verwenden. Die Bromide kommen vorteilhaft in Gestalt ihrer Erdalkalisalze und insbesondere ihrer Alkalisalze in Frage, z.B. Calciumbromid, Magnesiumbromid, Lithiumbromid und insbesondere Natriumbromid und Kaliumbromid.

Als Oxidationsmittel verwendet man zweckmässig: Chromverbindungen, wie Kalium-, Natrium-, Ammoniumbichromat, Chromsäure, Chromylchlorid; Permanganate wie Kaliumpermanganat oder $MnO_2$; Salpetersäure und ihre Salze, z.B. Natrium-nitrat, Silbernitrat, Kalium-, Natrium-nitrat, Lithium-, Calcium-, Magnesium-, Nickel-, Chrom-, Kupfer-, Kobalt-, Cer-, Thorium-, Wismuth-, Eisen-, Quecksilber-nitrat; Metalloxide wie PbO, $PbO_2$, CuO, $Os_5O_4$, $RuO_4$, HgO, $SeO_2$, $Ag_2O$; Metallsalze wie Eisen-III-chlorid, Eisen-IIIcyanide wie Kaliumhexacyanoferrat, Kupfersalze wie das Chlorid, Sulfat, Acetat, Bleitetraacetat, Mangantetraacetat, Quecksilber-II-acetat, Silberchlorat; organische Oxidationsmittel wie Chloranil, Kaliumnitrosodisulfonat, p-Nitrosodimethylanilin; Sauerstoff, Ozon. Die Oxidationsmittel werden vorteilhaft in einem Verhältnis von 0,001 bis 0,5, vorzugsweise von 0,05 bis 0,1 Mol je Mol Ausgangsstoff II, im Falle von Sauerstoff als Oxidationsmittel in einem Verhältnis von 0,05 bis 2 Mol je Mol Ausgangsstoff II, verwendet. In einer bevorzugten Ausführungsform wird mit Wasserstoffperoxid als Oxidationsmittel, zweckmässig in einer Menge von dem 1- bis 1,5-fachen, insbesondere dem 1- bis 1,25-fachen Äquivalentgewicht, bezogen auf Ausgangsstoff II, halogeniert. Das Wasserstoffperoxid wird zweckmässig in Form seiner 5- bis 60-, vorzugsweise 10- bis 30-gewichtsprozentigen, wässrigen Lösung verwendet. Gegebenenfalls kommen auch Stoffe in Betracht, die unter den Umsetzungsbedingungen Wasserstoffperoxid bilden, z.B. anorganische oder organische Peroxoverbindungen wie Natriumperoxid, Kaliumperoxid, Magnesiumperoxid, Calciumperoxid, Zinkperoxid, Bariumperoxid, Bariumsuperoxid; Hydroperoxide wie $NaOOH \cdot 0,5\ H_2O_2$, $NH_4OOH$; entsprechende Hydrate wie $CaO_2 \cdot 8H_2O$, Peroxohydrate wie $BaO_2 \cdot H_2O_2$ und $BaO_2 \cdot 2H_2O_2$; Peroxodischwefelsäure und Peroxomonoschwefelsäure und ihre Salze wie Natriumperoxodisulfat, Kaliumperoxodisulfat, Ammoniumperoxodisulfat; Peroxocarbonate wie Natriumperoxocarbonat und Calciumperoxocarbonat; Peroxophosphat wie das Kaliumperoxodiphosphat.

Ebenfalls kommen Bromierungsmittel wie Säurebromide der Phosphorsäure, phosphorigen Säure, Kohlensäure, Oxalsäure, Schwefelsäure, schwefligen Säure, z.B. Sulfurylbromid, Thionylbromid, Phosphorpentabromid, Phosphortribromid, Phosphoroxybromid, Oxalylbromid, Bromsulfonsäure, Kohlensäuredibromid in Frage. Weiterhin kommen in einzelnen Fällen Halogensauerstoffsäuren, ihre Anhydride oder ihre Salze, z.B. hypobromige Säure, Bromsäure, ihre Natrium- und Kaliumsalze, in Betracht. Ebenfalls sind N-Halogenderivate organischer Säureamide, z.B. Brom-N-succinimid, Brom-N-glutarimid, Brom-N-adipinsäureimid, Bromsulfaminsäure, Bromsulfamid, und die Bromderivate von Cyanursäure, 5,5-Dimethylhydantoin, geeignet. Gegebenenfalls können die vorgenannten Halogenverbindungen auch zusammen mit Oxidationsmitteln und/oder freien Halogenen verwendet werden.

Bevorzugt sind Bromsuccinimid, N-Bromacetamid, Perbromide wie Pyridiniumperbromid, 2,4,4,6-Tetrabromcyclohexa-2,5-dienon, 5,5-Dibrom-2,2-dimethyl-4,6-dioxo-dioxan-1,3 und insbesondere Brom.

Die Umsetzung wird bei einer Temperatur von 20 bis 100°C, vorzugsweise von 35 bis 90°C, insbesondere von 40 bis 70°C, mit Unterdruck, Überdruck oder drucklos, diskontinuierlich oder kontinuierlich, durchgeführt. Zweckmässig verwendet man unter den Reaktionsbedingungen inerte, organische Lösungsmittel. Als Lösungsmittel kommen z.B. in Frage: aromatische Kohlenwasserstoffe, z.B. Toluol, Äthylbenzol, o-, m-, p-Xylol, Isopropylbenzol, Methylnaphthalin; Halogenkohlenwasserstoffe, insbesondere Brom- und Chlorkohlenwasserstoffe, z.B. Tetrachlorethylen, 1,1,2,2- oder 1,1,1,2-Tretrachlorethan, Amylchlorid, Cyclohexylchlorid, 1,2-Dichlorpropan, Methylenchlorid, Dichlorbutan, Isopropylbromid, n-Propylbromid, Butylbromid, Chloroform, Bromoform, Ethyljodid, Propyljodid, Chlornaphthalin, Dichlornaphthalin, Tetrachlorkohlenstoff, 1,1,1- oder 1,1,2-Trichlorethan, Trichlorethylen, Pentachlorethan, 1,2-Dichlorethan, 1,1-Dichlorethan, n-Propylchlorid, 1,2-cis-Dichlorethylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan; und entsprechende Gemische. Zweckmässig verwendet man das Lösungsmittel in einer Menge von 80 bis

10000 Gewichtsprozent, vorzugsweise von 100 bis 600 Gewichtsprozent, bezogen auf Ausgangsstoff II.

Die Reaktion kann wie folgt durchgeführt werden: Ein Gemisch von Ausgangsstoff II und Bromierungsmittel wird während 1 bis 12 Stunden bei der Reaktionstemperatur umgesetzt. Der Endstoff wird dann in üblicher Weise, z.B. durch Wäsche des Gemischs und fraktionierte Destillation, isoliert.

Die so herstellbaren α-Bromacetophenonoximether I sind wertvolle Ausgangsstoffe für die Herstellung von Pharmazeutika, Farbstoffen und Schädlingsbekämpfungsmitteln. So sind die α-Bromacetophenonoximether der Formel I wertvolle Zwischenprodukte für die Synthese von fungizid wirksamen α-Acetophenonoximethern der Formel III

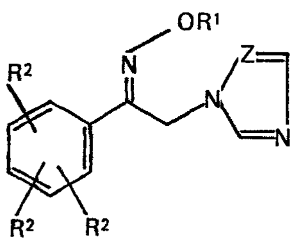

III,

in der Z für CH oder N steht und $R^1$ und $R^2$ die in Formel I angegebenen Bedeutungen haben. Die fungiziden Wirkstoffe der Formel III sind Gegenstand der DE-OS 2723942 und der DE-OS 2657578. Für ihre Herstellung wird die Umsetzung der entsprechenden Phenacylchloride oder -bromide (Halogenketone) mit den Azolen gemäss DE-OS 2431407 und die anschliessende Oximierung und Verätherung empfohlen. Demgegenüber führt die Umsetzung der aus den gut zugänglichen α-Bromacetophenonoximether der Formel I mit den Azolen zu befriedigenderen Ausbeuten, abgesehen davon, dass die α-Bromacetophenonoximether für diesen Zweck nicht weiter gereinigt zu werden brauchen, das heisst, sie können ohne weitere Reinigung mit 1,2,4-Triazol oder Imidazol zu den fungiziden Wirkstoffen der Formel III umgesetzt werden.

Die Umsetzung wird bei einer Temperatur im Bereich zwischen 20 und 120°C in einem inerten, organischen Lösungsmittel durchgeführt. Geeignet sind Alkohole, wie Methanol oder Ethanol, Nitrile, wie Acetonitril, Amide, wie Dimethylformamid, Dimethylacetamid. Auch Gemische dieser Lösungsmittel können verwendet werden.

Zur Bindung des bei der Umsetzung entstehenden Bromwasserstoffs wird dem Reaktionsgemisch eine basische Substanz, beispielsweise ein Alkalimetallcarbonat, wie Natriumcarbonat oder Kaliumcarbonat, ein Alkoholat, wie Natriummethylat, Natriumethylat, Natriumhydrid, Butyllithium, an Amin, wie Diisopropylethylamin, ein Lithiumamid, wie Lithiumdiisopropylamid, zugegeben. Der Bromwasserstoff kann auch durch einen Überschuss an Imidazol oder 1,2,4-Triazol gebunden werden.

Die Reaktionspartner werden zweckmässig in stöchiometrischen Mengen eingesetzt. Vorzugsweise arbeitet man mit einem Überschuss an 1,2,4-Triazol bzw. Imidazol von 20 bis 200 Molprozent.

Herstellung von α-(1,2,4-Triazolyl)-acetophenonoxim-O-(4-chlorbenzyl)-ethernitrat.

Ein Gemisch von 0,2 mol 1,2,4-Triazol und 0,2 Mol Natriumcarbonat in 200 ml Ethanol wird zum Rückfluss erhitzt. Dann gibt man eine Lösung von 0,1 Mol α-Bromacetophenonoxim-O-(4-chlorbenzyl)-ether in 50 ml Ethanol zu. Nach drei Stunden Kochen unter Rückfluss wird eingeengt und mit Dichlormethan/Wasser aufgenommen. Die organische Phase wird mit Wasser gelöst. Unter Kühlung wird Salpetersäure zugetropft. Das ausgefallene Nitrat wird abgesaugt und getrocknet. Man erhält 15 g (= 39%) -(1,2,4-Triazolyl)-acetophenonoxim-O-(4-chlorbenzyl)-ethernitrat vom Schmp. 130°C (Zers.).

Die in den folgenden Beispielen aufgeführten Teile bedeuten Gewichtsteile. Sie verhalten sich zu den Volumenteilen wie Kilogramm zu Liter.

Beispiel 1

14,7 Teile 2,4-Dichloracetophenonoxim-O-benzylether werden in 100 Teilen Tetrachlormethan vorgelegt und bei 50°C unter Lichtausschluss eine Lösung von 8 Teilen Brom in 50 Teile Tetrachlormethan zugegeben. Nach 2 Stunden Rühren wird das Gemisch zweimal mit 100 Teilen Wasser und einmal mit 100 Teilen Natriumhydrogenacarbonat-Lösung gewaschen und über Kaliumcarbonat getrocknet. Nach fraktionierter Destillation erhält man 13,1 Teile α-Brom-2,4-dichloracetophenonoxim-O-benzylether (70% der Theorie) vom Kp 120°C ($6\cdot10^{-5}$ mbar).

Beispiel 2

15,2 Teile 4-Bromacetophenonoxim-O-benzylether werden in 100 Teilen Dichlormethan vorgelegt und bei 40°C unter Lichtausschluss eine Lösung von 8 Teilen Brom in 50 Teile Dichlormethan zugegeben. Nach 4 Stunden Rühren wird das Gemisch analog Beispiel 1 aufgearbeitet. Man erhält 13,7 Teile α-4-Dibromacetophenonoxim-O-benzylether (72% der Theorie) vom Kp 110°C ($8\cdot10^{-5}$ mbar).

**Patentanspruch**

Verfahren zur Herstellung von α-Bromacetophenonoximethern der Formel

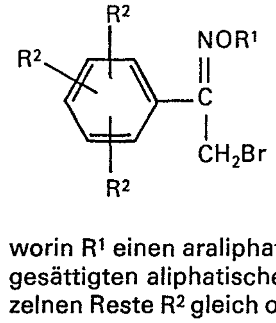

I,

worin $R^1$ einen araliphatischen oder zweifach ungesättigten aliphatischen Rest bedeutet, die einzelnen Reste $R^2$ gleich oder verschieden sein können und jeweils ein Wasserstoffatom, ein Halogenatom oder einen aliphatischen Rest bezeichnen, dadurch gekennzeichnet, dass man Acetophenonoximether der Formel

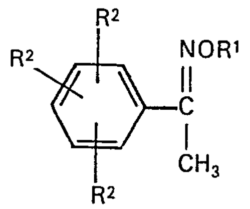

II,

worin $R^1$ und $R^2$ die vorgenannte Bedeutung besitzen,mit einem Bromierungsmittel bei einer Temperatur von 20 bis 100°C unter Lichtausschluss umsetzt.

**Claim**

A process for the preparation of α-bromoacetophenone-oxime-ethers of the formula

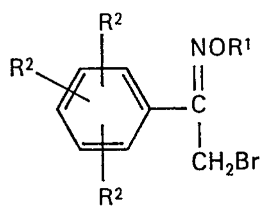

I,

where $R^1$ is an araliphatic or a doubly unsaturated aliphatic radical and $R^2$ is hydrogen, halogen or an aliphatic radical, the individual $R^{2\prime}$s being identical or different, wherein an acetophenone-oxime-ether of the formula

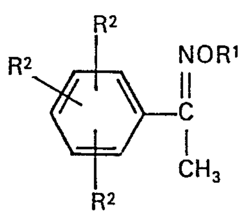

II,

where $R^1$ and $R^2$ have the above meanings, is reacted with a brominating agent at from 20 to 100°C, in the absense of light.

**Revendication**

Procédé de préparation d'éthers d'alpha-bromacétophéno-noximes de formule

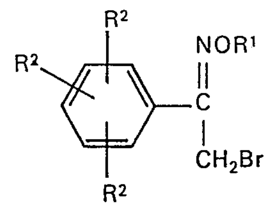

I,

dans laquelle $R^1$ représente un reste araliphatique ou aliphatique di-insaturé, les divers symboles $R^2$, qui peuvent être identiques ou différents, représentent chacun un atome d'hydrogène, un atome d'halogène ou un reste aliphatique, caractérisé en ce que l'on fait réagir des éthers d'acétophénonoximes de formule

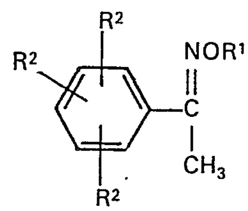

II,

dans laquelle $R^1$ et $R^2$ ont les significations indiquées ci-dessus, avec un agent bromant à une température de 20 à 100°C et à l'abri de la lumière.